# EUROPEAN PATENT SPECIFICATION

(11) **EP 1 162 945 B1**
(45) Date of publication and mention of the grant of the patent: **06.08.2003**
(21) Application number: 00912780.4
(22) Date of filing: 23.03.2000
(51) Int. Cl.: A61K 9/00, A61K 9/16, A61P 37/04, A61K 39/02, A61K 39/39

(54) **PARTICLE BASED VACCINE COMPOSITION**
IMPFSTOFFZUSAMMENSETZUNG AUF BASIS VON PARTIKELN
COMPOSITION VACCINALE A BASE DE PARTICULES

(30) Priority: 24.03.1999 GB 9906695
(43) Date of publication of application: 19.12.2001
(73) Proprietor: The Secretary of State of Defence, Salisbury, Wiltshire SP4 0JQ (GB)
(72) Inventor: ALPAR, Hazire Oya, Aston Triangle, Birmingham B4 7ET (GB); WILLIAMSON, Ethel, Diane, Salisbury,Wiltshire SP4 0JQ (GB); BAILLIE, Leslie William James, Salisbury, Wiltshire SP4 0JQ (GB)
(74) Representative: Skelton, Stephen Richard
(86) International application number: GB0001108
(87) International publication number: WO00056282

(56) References cited:
- WO-A-94/15636
- WO-A-97/28263
- US-A- 4 372 949
- O'HAGAN D T ET AL: "Intravaginal immunization in sheep using a bioadhesive microsphere antigen delivery system." VACCINE, vol. 11, no. 6, 1993, pages 660-664, XP002141394 ISSN: 0264-410X
- DATABASE CHEMABS [Online] CHEMICAL ABSTRACTS SERVICE, COLUMBUS, OHIO, US; STURESSON, C. ET AL: "Preparation and characterization of hydrophobic PLG microspheres suitable for oral vaccination with rotavirus" retrieved from STN Database accession no. 125:230587 XP002141396 & PROC. INT. SYMP. CONTROLLED RELEASE BIOACT. MATER. (1996), 23RD, 515-516 ,
- ALONSO MARIA J ET AL: "Determinants of release rate of tetanus vaccine from polyester microspheres." PHARMACEUTICAL RESEARCH (NEW YORK), vol. 10, no. 7, 1993, pages 945-953, XP000915016 ISSN: 0724-8741
- O'HAGAN D T ET AL: "Vaginal immunization of rats with a synthetic peptide from human immunodeficiency virus envelope glycoprotein." JOURNAL OF GENERAL VIROLOGY, (1992 AUG) 73 ( PT 8) 2141-5. , XP002141395

## Description

The present invention relates to compounds for use as immunostimulants, as well as to a composition which is useful for delivering medicaments and particularly vaccines, in particular to mucosal surfaces, for example in oral formulations. The invention further comprises methods of treating individuals using the composition and methods of preparing the composition.

A prime objective in the field of vaccination is the development of a non-parenteral immunisation regimen, which facilitate induction of comparable levels of systemic immunity to that elicited by conventional sub-cutaneous and intra-muscular injections.

The nasapharyngeal passages and pulmonary regions of the respiratory tract represent potential targets for the systemic delivery of peptidergic drugs and vaccines. The relative ease with which therapeutic agents can be inhaled, or introduced into the nose, make these modes of immunisation attractive in terms of probable patient compliance. Furthermore, respiratory mucosae offer certain morphological, physiological and immunological advantages over other non-parenteral sites in terms of immunisation, particularly against pathogenic entitities which affect or utilise mucosal surfaces as portals of entry. This is because effective vaccination against these pathogens normally requires mucosae to the adequately protected with locally produced antibodies of the secretory IgA (sIgA) isotype. Whilst mucosal surfaces are usually poorly protected with IgA following parenteral administration of vaccines, it is now apparent that successful delivery of antigenic material to immunoresponsive elements in mucosa-associated lymphoid tissue (MALT) can result in vigorous stimulation of the mucosal arm of the immune system. By means of the common mucosal immune system (CMIS) it is feasible that several anotomically disparate mucosal surfaces could be protected through mucosal administration of a vaccine at a single site. Mucosal vaccination offers the added advantage that some degree of systemic immunity can be induced in concert with local responses due to translocation of antigenic material from sub-epithelial compartments to systemic immunoresponsive tissues such as the spleen.

Despite the logistical and immunological factors which favour non-parenteral immunisation, simple mucosal application of antigenic proteins, for example in the gastrointestinal or respiratory tracts, is usually ineffectual in terms of vaccination. Enzymatic or chemical destruction, combined with poor absorption into sub-epithelial compartments dictate that mucosally administered vaccines usually require some form of adjuvant or delivery vehicle. One approach is to encapsulate antigenic material within microparticulate polymeric carriers, such as poly-DL-lactide (PLA) microspheres (Vaccine 1994, 12, 5-11). Such procedures serve to protect labile vaccines from lumenal degradation and enhance absorption into mucosal and systemic compartments (J.H. Eldridge et al., Seminars in Hematology, (1993), 30, 16-25). There is good evidence that microencapsulation may also adjuvantise by converting soluble antigenic molecules into particulate species, thus promoting vaccine uptake into antigen presenting cells (APC)(Y. Tabata et al., Adv. Polym. Sci. (1990), 94, 107-141, L. Vidard et al., J. Immunol. (1996), 156, 2809-2818, N. Van Rooijen, Immunol. Today (1990) 11, 436-439) or microfold cells (M-cells) in lymphoid follicles (R.I. Walker et al., Vaccine, 12, 387, 1994, D.T. O'Hagan et al,, Vaccine, 1989, 7, 421-424, P.G. Jenkins et al., J. Drug Targetting, 1995, 3, 79-81). Nasal delivery of microsphere formulation of vaccine has also been described (A.J. Almeida et al., J. Pharm & Pharmacology, 25, 198-203 1993, H.O. Alpar et al., J. Drug Targeting 2/2, 147-149, 1994, A.J. Almeida et al., J. Drug Targeting 3(b), 255-467 1996).

WO 91/06282 describes certain drug compositions which are suitable for intranasal delivery and which include absorption enhancers. Examples of suitable enhancers are lysophosphatidylcholine.

WO 94/15636 describes delayed release vaccine preparations intended for administration by the parenteral route, or by use of an implant. It does not disclose any compositions suitable for administration to a mucosal surface.

O'Hagan *et al.,* "Intravaginal immunisation in sheep using a bioadhesive microsphere antigen delivery system" (Vaccine, vol. 11, no. 6, 1993, pages 660-664, XP002141394) describes a formulation in which lysophosphatidylcholine is added to a preformed powder consisting of degradable starch microspheres. The lysophosphatidylcholine is not comprised within the microparticles. It is intimately mixed but not physically attached to the degradable starch microspheres.

Sturesson, C *et al.* (Database Chemabs Chemical Abstracts Service Columbus, Ohio, US) teaches a method for encapsulating fixed rotavirus particles in poly(lactide-co-glycolide) microspheres containing phosphatidylcholine (otherwise known as lecithin) for oral administration. The organic phase used in the preparation of the emulsions used in the formation of microspheres contains very small amounts (0.1 or 0.5% w/v) of phosphatidyl-choline (PC) as an emulsifier. This phase is further diluted significantly. As a result, the final concentration of the PC in the particles is extremely low, and would not provide the immunostimulant effect required by the amended claims.

(Alonso *et al*). describes a formulation, which is administered subcutaneously. Thus, it is not capable of being administered to a mucosal surface as required by the claims of the present application. In addition, the phospholipid used in the preparation is used in very low amounts (200 µl of a 2mg/ml solution) as an emulsifier only, and not as an immunostimulant.

The applicants have found that the presence of a phospholipid in a vaccine formulation has an immunostimulant effect, over and above that which could be explained by absorption enhancer effects.

Thus the invention provides a phospholipid for use as an immunostimulant.

As used herein, the term "immunostimulant" refers to an adjuvant which stimulates the immune system of a host animal to which it is administered and thereby increases the protective effect produced by a protective antigen administered to that animal, as compared to the effect which would be produced by administration of the protective antigen alone.

In particular, it has been noted that a microencapsulated biologically active formulation, especially of immunogens, which comprises a phospholipid in addition to the polymeric material used in the formation of the microparticles, has an increased biological effect. This may be particularly noticed when the formulation is administered by any route including parenteral or other routes, but is particularly effectively when administered by way of a mucosal surface.

Thus the invention provides a pharmaceutical composition, which comprises microparticles comprising
(i) biologically active agent which is capable of generating an immune response in an animal to which it is administered;
(ii) a polymeric material capable of forming microspheres ; and
(iii) an immunostimulant comprising a phospholipid.

In one embodiment, the composition is suitable for parenteral administration. A particular example is intramuscular (i.m.) administration.

In a preferred embodiment, the composition is suitable for non-parenteral administration for example to mucosal surfaces.

Administration to mucosal surfaces may be effected by oral application, by pulmonary application, for example by intratracheal administration, or particularly by intra-nasal application. In particular, the compositions of the invention are administered by the oral route.

The polymeric material used in the compositions of the invention is suitable for forming microparticles (sometimes known as microcapsules or microspheres). It may be a low, medium or high molecular weight polymer. Examples of low molecular weight polymers are polymers which have a molecular weight of between 0.1 and 10kDa, more preferably between 1 and 5 kDa and typically about 2-3kDA.

The use of high molecular weight polymers in the encapsulation of a tetanus vaccine for intramuscular administration has been described (Vaccine 1994, 12, 4, 299-306). A formulation of microencapsulated ricin toxoid vaccine which is applied intranasally has also been described (Vaccine 1994, 14, 11 1031). However, in that case, high molecular weight polymer microparticles (94kDa) were less effective than those prepared from a copolymer of lower molecular weight (72kDa).

The polymeric material used in the composition of the present invention suitably has a high molecular weight in excess of 94kDa, for example of 100kDa or more.

A particularly suitable polymeric material for use in the compositions of the invention comprises a poly(hydroxy)acid or or a copolymer thereof. A particular example is poly-(L-lactide) or PLA but other high molecular weight polymeric material such as poly(lactic/glycolic acid), polycyonacrylates, polyanhydrides, polycarbonates or polycaprolactones as are known in the art may be employed.

Examples of suitable phospholipids for use in the microparticles of the compositions of the invention include many pharmaceutically acceptable phospholipids or precursors therefore, which may be cationic, anionic or neutral in character. These include lecithin or its precursor phosphoryl choline, distearylphosphatidylcholine (DSPC) and phosphatidinylserine (PS). Examples of positively charged lipids include dipalmitoylphosphatidylcholine (DPPC) and dioleoyltrimethylammoniumpropane (DOTAP). A particularly preferred phospholipid is lecithin which is widely available and commonly used in other types of pharmaceutical composition.

Suitably the phospholipid is added to the composition in an amount of from 0.1% to 20%w/w, and preferably about 5%w/w.

The microparticles may optionally further comprise agents which stabilise emulsions such as polyvinylalcohol, methyl cellulose or dextrans.

They will suitably be of an average size of from 0.1µm to 10µm in diameter.

These compositions may be used to deliver a biologically active agents which are capable of generating a protective immune response in an animal, particularly a mammal, to which it is administered. Examples of such agents include antigenic polypeptides as well as nucleic acid sequences which may encode these polypeptides and which are known as "naked DNA" vaccines.

As used herein the expression "polypeptide" encompasses proteins or epitopic fragments thereof.

Suitable polypeptides are sub-unit vaccines or others such as tetanus toxoid, diptheria toxoid and *Bacillus anthracis* protective antigen (PA).

In one embodiment, the composition of the invention comprises a biologically active agent which is capable of generating a protective immune response against *Yersinia pestis*. The agent is suitably a sub-unit vaccine, for example as described in WO 96/28551. The vaccine described and claimed there comprises a combination of the V antigen of *Y. pestis* or an immunologically active fragment thereof or a variant of these, and the F1 antigen of *Y. pestis* or an immunologically active fragment thereof or a variant of these.

As used herein, the term "fragment" refers to a portion of the basic sequence which includes at least one antigenic determinant. These may be deletion mutants. One or more epitopic region of the sequence may be joined together.

The expression "variant" refers to sequences of nucleic acids which differ from the base sequence from which they are derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type.
Broadly speaking, fewer non-conservative substitutions will be possible without altering the biological activity of the polypeptide. Suitably variants will be at least 60% homologous, preferably at least 75% homologous, and more preferably at least 90% homologous to the base sequence. Homology in this instance can be judged for example using the algorithm of Lipman-Pearson, with Ktuple:2, gap penalty:4, Gap Length Penalty:12, standard PAM scoring matrix (Lipman, D.J. and Pearson, W.R., Rapid and Sensitive Protein Similarity Searches, *Science*, 1985, vol. 227, 1435-1441).

Preferably, vaccine compositions will further comprise a conventional adjuvant in order to increase or enhance the immune response to the biologically active material administered. Suitable adjuvants include pharmaceutically acceptable adjuvants such as Freund's incomplete adjuvant, alhydrogel, aluminium compounds and, preferably adjuvants which are known to up-regulate mucosal responses such as CTB, the non-toxic pentameric B subunit of cholera toxin (CT).

They may also comprise other known composition components such as colouring agents and preservatives and in particular cetrimide. These are suitably present in amounts of from 0.1 to 0.7%w/v.

In a particular embodiment, the microspheres used in the compositions may further comprise an S-layer proteins, in particular, S-layer proteins derived from a bacteria against which the biologically active agent produces a protective immune response. These proteins are suitably coated onto the surface of the particles. It has been shown (Sleyr et al., Crystalline bacterial cell surface proteins. Biotechnology Intelligence Unit, 1996, R.G. Landes Company and Academic Press Inc.) that the stability of liposomes can be increased by such coatings. S-layer proteins are found on the surface of most bacteria and form a regular two dimensional array known as an S-layer. Isolated S-layer proteins are able to form entropy driven monomolecular arrays in suspension, and on the surface of structures such as liposomes.

Compositions of the invention may be suitable for oral and intranasal application. They are particularly effective when applied orally.

They may comprise microparticles per se which are optionally preserved, for example by lyophilisation, or the microparticles may be combined with a pharmaceutically acceptable carrier or excipient. Examples of suitable carriers include solid or liquid carriers as is understood in the art.

The invention further provides a method of producing a pharmaceutical composition, which method comprises encapsulating a biologically active agent as described above in a polymeric material which suitably has a high molecular weight and in particular a molecular weight of 100kDa or more, in the presence of a phospholipid such as lecithin. The phospholipid may be incorporated within the microparticle, or at the surface, of preferably is distributed throughout the microparticle.

Methods of forming liposomes are well known in the art. They include dispersion of dehydrated lipid films into an aqueous media, emulsion techniques and lyophilisation methods as are well known in the art.

Microparticles of the invention are suitably prepared using a double emulsion solvent evaporation method. Briefly, the biologically active agent, in solution or in a suitably lyophilised state, is suspended or dissolved in an aqueous solution of the polymeric material such as polyvinyl alcohol (PVA) and the phospholipid such as lecithin. A solution of the polymer in an organic solvent such as dichloromethane, is added with vigorous mixing. The resultant emulsion is then dropped into a secondary aqueous phase, also containing polymeric (PVA or the like) and optionally also the phospholipid with vigorous stirring. After addition, the organic solvent is allowed to evaporate off and the resultant microspheres separated. appropriate dosage unit of the active agent. This will vary depending upon the nature of the active agent being employed, the nature of the patient, the condition being treated and other clinical factors. In general however, the composition of the invention will comprise approximately 0.1 to 10 wt% of active ingredient.

The amount of polymer in the composition will be of the order of 70 to 99wt% of the composition, and suitably from 90 to 99wt% of the microparticle components will be the polymeric material. The amount of phospholipid, will be of the order of 0.1 to 10 wt % of the composition. Preferably, the ratio of the polymeric material to the phospholipid is from 99:1 to 9:1% w/w.

The compositions of the present invention preferably comprise prophylactic or therapeutic vaccines.

In use, a reasonable dosage for nasal administration would be of from 0.05g to 0.2g. A formulation for oral administration to humans will generally contain, for example, from 0.5 mg to 2 g of active agent within a composition as defined above.

Preferred compositions of the inventions are vaccine compositions where the biologically active agent is able to produce a protective immune response to a pathogenic organism as described above. Thus, in a further aspect, the invention provides a method of protecting a mammal against infection, which method comprises administration of a vaccine composition as described above to mammal. In particular, the composition is applied to a mucosal surface, in particular gastrointestinal surface, of a mammal.

The applicants have shown that through the oral administration of tetanus toxoid in accordance with the present invention, antibody titres were achieved which are in excess of those associated with protection from the relevant toxin, and protection to a subcutaneous challenge of tetanus toxoid was provided.

A further aspect of the invention comprises the use of a phospholipid as an immunostimulant in the production of a vaccine for use in prophylactic or therapeutic treatment.

The invention will now be particularly described by way of example with reference to the accompanying drawings in which:
Figure 1 illustrates the serum immune response in mice to orally delivered microencapsulated and free tetanus toxoid with 50 lf units on day 1, 3, 5 and boosted on day 28, 30 and 50;
Figure 2 illustrates the serum immune immune response after 86 days; and
Figure 3 illustrates the serum immune immune response after 162 days following a subcutaneous "mock challenge" with tetanus toxoid.

### Example 1

### Microencapsulation of Tetanus toxoid

Poly-L-lactide of molecular weight 100kDa (Polysciences Inc. USA) was used in a modification of the double emulsion solvent evaporation method (Y. Ogawa et al., Chem. Pharm. Bull., 36 (1988) 1095-1103). Briefly, 1.5ml of a 1.5% w/v aqueous solution of polyvinyl alcohol (PVA)(13-25k) containing tetanus toxoid (4000Lf units) was prepared. This formed the aqueous phase. An organic phase was prepared separately by mixing with 200mg of 100K PLA polymer dissolved in 5ml of HPLC grade dichloromethane (DCM) with 5%w/w lecithin. The two phases were homogenised together using a Silverson homogeniser (Silverson, UK) for 1 minute. The resultant primary emulsion was added, drop by drop, into a secondary aqueous phase (75ml) comprising 1.5%w/v PVA and 0.1%w/v lecithin. The mixture was then homogenised using a Silverson homogeniser for 5 minutes. This secondary phase was gently stirred overnight to until the dichloromethane had evaporated. Microspheres were recovered by centrifugation, washed with double distilled water three times and then lyophilised.

Other microspheres using 10%w/w lecithin instead of the 5%w/w solution were prepared using a similar procedure (10% LEC PLA MS). Further microspheres where the lecithin was omitted entirely from the preparation were also prepared by an analgous method but 5% w/w lecithin was used instead of water alone for the oral administration of microspheres (PLA MS in 5% LEC).

In addition, this method was used to prepare comparative microspheres where the lecithin in the primary emulsion was replaced by either 5% w/w or 10%w/w saponin (SAP) or 10% w/w or 20% w/w stearyl amine (SA) and lecithin omitted from the preparation entirely. Finally, PLA microspheres without any adjuvant were prepared.

### Example 2

### Immunisation Study

Groups of three Balb/c female mice were orally dosed with the microspheres prepared in Example 1 containing 50LF tetanus toxoid on days 1, 3 and 5 of the trial, and boosted on day 28 and 30. Another group of mice was orally dosed with similar amounts of tetanus toxoid but in free solution.

Serum immune responses were monitored. Tail vein blood samples were taken from all animals on days 14 and 35 of the experiment. Titration of IgG antibody isotypes in serum samples was achieved using an ELISA. Briefly, individual serum samples were aliquoted to microtitre plates pre-coated with tetanus toxoid. Binding of serum antibody was detected with peroxidase-labelled secondary antibody to mouse IgG (Sigma A4416). Antibody titre was estimated as the maximum dilution of the serum giving an absorbance reading greater that the maximum optical density (OD) of titrated naïve serum. From this, mean titres ± standard deviation (SD) were derived per treatment group.

At day 162, the immune responses following a subcutaneous "mock challenge" with TT as monitored.

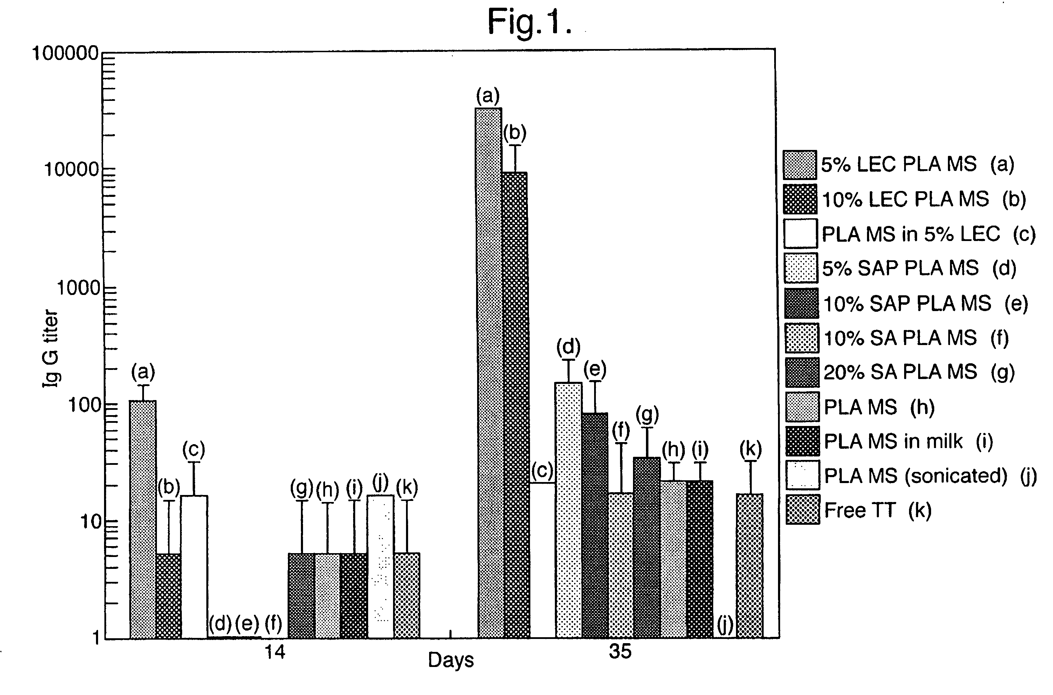

The results are shown in Figures 1-3. In these Figures, the following letters represent compositions prepared as described above as follows:
(a) 5% lecithin containing PLA microsphere composition:
(b) 10% lecithin containing PLA microsphere composition;
(c) PLA microspheres in the presence of 5% lecithin;
(d) 5% w/w saponin containing microsphere composition;
(e) 10%w/w saponin (SAP) containing microsphere composition;
(f) 10% w/w stearyl amine (SA) containing microsphere composition;
(g) 20% w/w stearyl amine (SA) containing microsphere composition;
(h) PLA microspheres;
(i) PLA microspheres in milk;
(j) PLA micropheres which have been sonicated;
(k) Free tetanus toxoid.

Microsphere formulations containing lecithin showed an amplification of > 3000 as compared to free tetanus toxoid and other microsphere formulations. Microspheres which contained no lipid in a phospholipid vehicle gave similar results to the free tetanus toxoid.

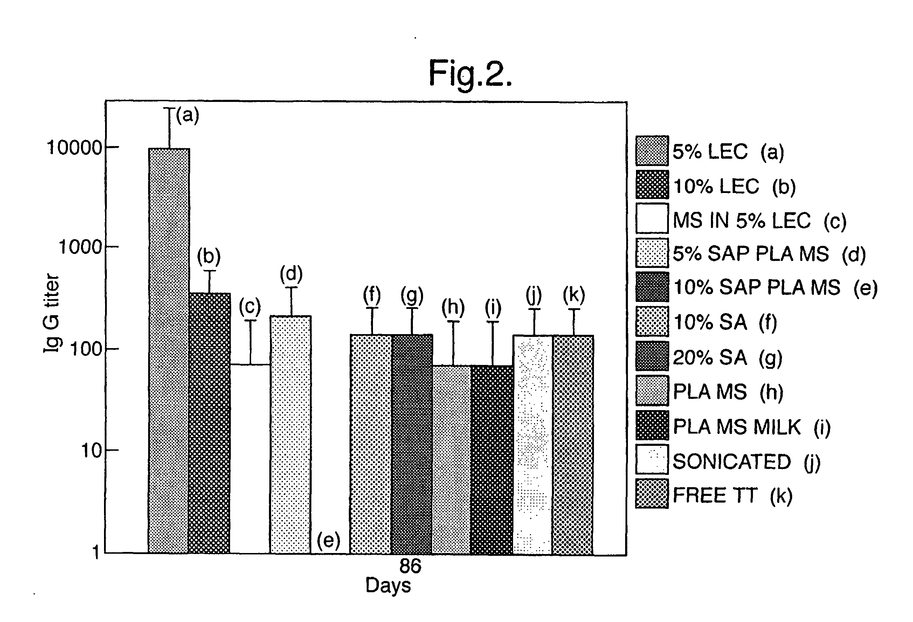

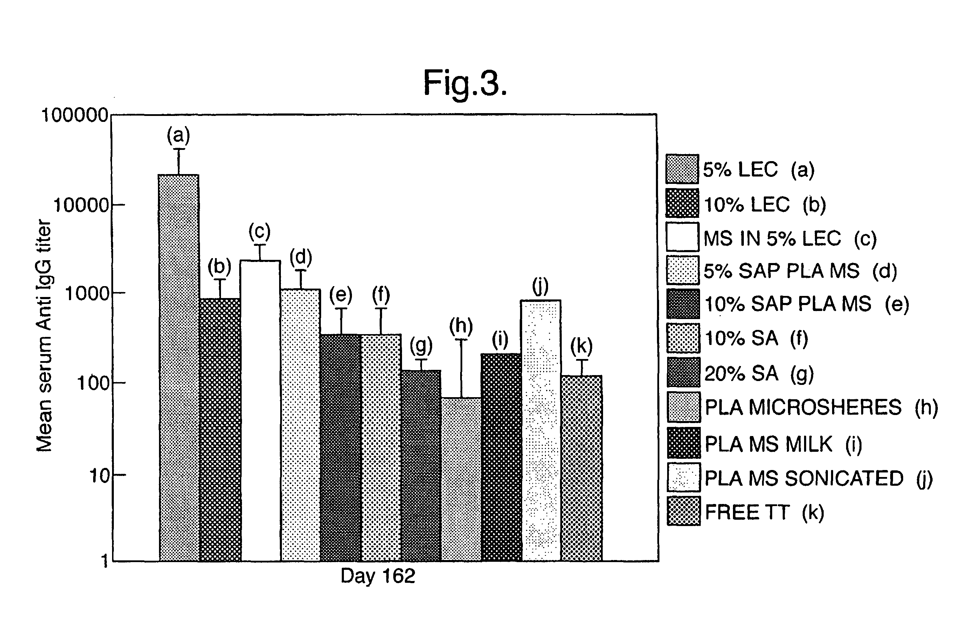

Even after 86 days of the dosing and following the s.c. challenge at day 162, titres were above the protective titre levels (Figures 2 and 3) when formulations in accordance with the invention were employed.

## Claims

1. A pharmaceutical composition which is adapted for administration to a mucosal surface, which comprises microspheres comprising
(i) a biologically active compound capable of generating an immune response;
(ii) a polymeric material capable of forming microspheres ; and
(iii) an immunostimulant comprising a phospholipid in an amount of from 0.1% to 20%w/w and wherein the ratio of the polymeric material to the phospholipid is from 99:1 to 9:1% w/w.

2. A composition according to claim 2 which is adapted for oral administration.

3. A composition according to claim 1 of claim 2 wherein the polymeric material has molecular weight in excess of 94 kDa.

4. A composition according to claim 3 wherein the polymeric material has a molecular weight of 100kDa or more.

5. A composition according to any one of the preceding claims wherein the polymeric material comprises a poly(hydroxy)acid or or a copolymer thereof.

6. A composition according to any one of the preceding claims wherein the phospholipid is selected from lecithin or its precursor phosphoryl choline, distearylphosphatidylcholine (DSPC) and phosphatidinylserine (PS).

7. A composition according to claim 6 wherein the phospholipid is lecithin.

8. A composition according to any one of the preceding claims wherein the amount of phospholipid is 5%w/w.

9. A composition according to any one of the preceding claims wherein the biologically active compound capable of generating an immune response in an animal to which it is administered comprises a polypeptide or a nucleic acid.

10. A composition according to claim 9 wherein the biologically active agent comprises tetanus toxoid, diptheria toxoid, *Bacillus anthracis* protective antigen (PA) or an agent which is capable of generating a protective immune response against *Yersinia pestis*.

11. A composition according to claim 9 or claim 10 which further comprises a conventional adjuvant in order to increase the immune response to the biologically active material administered.

12. A composition according to any one of the preceding claims which further comprises a preservative.

13. A composition according to claim 12 wherein the preservative is cetrimide and this is present in amounts of from 0.1 to 0.7%w/v.

14. A composition according to any one of the preceding claims wherein the microspheres used in the compositions may further comprise an S-layer protein.

15. A composition according to claim 14 wherein the biologically active agent is as defined in claim 9 and wherein the S-layer proteins are derived from a bacteria against which the biologically active agent produces a protective immune response.

16. A composition according to any one of the preceding claims wherein the microspheres are suspended in a diluent or carrier.

17. A method of producing a pharmaceutical composition according to claim 1, which method comprises encapsulating a biologically active agent which is capable of generating a protective immune response, in a polymeric material, in the presence of a phospholipid in an amount of 0.1% to 20% w/w, wherein the ratio of the polymeric material to the phospholipid is from 99:1 to 9:1% w/w.

18. A method according to claim 17 wherein the biologically active agent, in solution or in a suitably lyophilised state, is suspended or dissolved in an aqueous solution of the polymeric material and the phospholipid, a solution of the polymer in an organic solvent is added with vigorous mixing, the resultant emulsion is dropped into a secondary aqueous phase which contains a polymeric material with vigorous stirring, after which the organic solvent is allowed to evaporate off and the resultant microspheres separated.

19. A method according to claim 18 wherein the secondary aqueous phase also comprises phospholipid.

20. A method according to any one of claims 17 to 19 wherein the phospholipid is distributed throughout the microsphere.

21. A method according to any one of claims 17 to 20 wherein the composition comprises a prophylactic or therapeutic vaccine.

22. A composition according to any one of claims 1 to 16 for use in a method of protecting a mammal against infection by a pathogen.

23. A composition according to claim 22 wherein the composition is applied to a mucosal surface of said mammal.

24. A composition according to claim 23 wherein the mucosal surface comprises a gastrointestinal surface.

25. The use of a phospholipid in the production of a microsphere for use in the composition according to claim 1.

26. The use of a phospholipid as an immunostimulant in the production of microspheres for use in a prophylactic or therapeutic vaccine for administration to a mucosal surface.

## Patentansprüche

1. Pharmazeutische Zusammensetzung, die für eine Verabreichung auf eine Schleimhautoberfläche geeignet ist und Mikroteilchen umfasst, die
(I) eine biologisch wirksame Verbindung, die in der Lage ist, eine Immunantwort hervorzurufen,
(II) ein polymeres Material, das in der Lage ist, Mikrokugeln zu bilden, und
(III) ein Immunstimulanz, das ein Phospholipid mit einem Anteil von 0,1 bis 20 % Gew./Gew. enthält, wobei das Verhältnis von polymerem Material zu Phospholipid 99 : 1 bis 9:1 % Gew./Gew. beträgt,
umfassen.

2. Zusammensetzung nach Anspruch 1, die für die orale Verabreichung geeignet ist.

3. Zusammensetzung nach Anspruch 1 oder Anspruch 2, wobei das Molekulargewicht des polymeren Materials mehr als 94 kDa beträgt.

4. Zusammensetzung nach Anspruch 3, wobei das Molekulargewicht des polymeren Materials 100 kDa oder mehr beträgt.

5. Zusammensetzung nach einem der vorhergehenden Ansprüche, wobei das polymere Material eine Poly(hydroxy)säure oder ein Copolymer davon umfasst.

6. Zusammensetzung nach einem der vorhergehenden Ansprüche, wobei das Phospholipid aus Lecithin oder dessen Vorläufer Phosphorylcholin, Distearylphosphatidylcholin (DSPC) und Phosphatidinylserin (PS) ausgewählt wird.

7. Zusammensetzung nach Anspruch 6, wobei das Phospolipid Lecithin ist.

8. Zusammensetzung nach einem der vorhergehenden Ansprüche, wobei der Anteil des Phospolipids 5 % Gew./Gew. beträgt.

9. Zusammensetzung nach einem der vorhergehenden Ansprüche, wobei die biologisch wirksame Verbindung, die in der Lage ist, bei einem Tier, welchem sie verabreicht worden ist, eine Immunantwort hervorzurufen, ein Polypeptid oder eine Nukleinsäure umfasst.

10. Zusammensetzung nach Anspruch 9, wobei das biologisch wirksame Mittel Tetanustoxoid, Diphtherietoxoid, vor *Bacillus anthracis* schützendes Antigen (PA) oder ein Antigen, das in der Lage ist, eine vor *Yersinia pestis* schützende Immunantwort hervorzurufen, umfasst.

11. Zusammensetzung nach Anspruch 9 oder Anspruch 10, die weiterhin einen herkömmlichen Zusatzstoff zur Verstärkung der Immunantwort auf das verabreichte biologisch wirksame Material umfasst.

12. Zusammensetzung nach einem der vorhergehenden Ansprüche, die außerdem einen Konservierungsstoff umfasst.

13. Zusammensetzung nach Anspruch 12, wobei der Zusatzstoff Cetrimid ist, das mit einem Anteil von 0,1 bis 0,7 % Gew./Vol. vorliegt.

14. Zusammensetzung nach einem der vorhergehenden Ansprüche, wobei die in den Zusammensetzungen verwendeten Mikrokugeln ferner ein S-Schicht-Protein umfassen können.

15. Zusammensetzung nach Anspruch 14, wobei das biologisch wirksame Mittel wie in Anspruch 9 definiert ist und die S-Schicht-Proteine von einem Bakterium stammen, gegen welches das biologisch wirksame Mittel eine schützende Immunantwort hervorruft.

16. Zusammensetzung nach einem der vorhergehenden Ansprüche, wobei die Mikrokugeln in einem Verdünnungsmittel oder Träger suspendiert sind.

17. Verfahren zur Herstellung einer pharmazeutischen Zusammensetzung nach Anspruch 1, welches das Einkapseln eines biologisch wirksamen Mittels, das in der Lage ist, eine schützende Immunantwort hervorzurufen, in einem polymeren Material in Gegenwart eines Phospholipids mit einem Anteil von 0,1 bis 20 Gew./Gew. umfasst, wobei das Verhältnis von polymerem Material zu Phospholipid 99 : 1 bis 9 : 1 % Gew./Gew. beträgt.

18. Verfahren nach Anspruch 17, wobei das biologisch wirksame Mittel, das sich in Lösung oder einem geeignet lyophilsierten Zustand befindet, in einer wässrigen Lösung des polymeren Materials und des Phospholipids suspendiert oder gelöst wird, unter starkem Rühren eine Lösung des Polymers in einem organischen Lösungsmittel zugegeben, die erhaltene Emulsion in eine sekundäre wässrige Phase, die ein polymeres Material enthält, unter starkem Rühren getropft und danach das organische Lösungsmittel verdunsten gelassen wird und die resultierenden Mikrokugeln abgetrennt werden.

19. Verfahren nach Anspruch 18, wobei die sekundäre wässrige Phase auch ein Phospholipid enthält.

20. Verfahren nach einem der Ansprüche 17 bis 19, wobei das Phospholipid in der gesamten Mikrokugel verteilt wird.

21. Verfahren nach einem der Ansprüche 17 bis 20, wobei die Zusammensetzung einen prophylaktischen oder therapeutischen Impfstoff umfasst.

22. Zusammensetzung nach einem der Ansprüche 1 bis 16 für die Verwendung in einem Verfahren zum Schutz eines Säugetiers vor einer Infektion durch ein Pathogen.

23. Zusammensetzung nach Anspruch 22, wobei die Zusammensetzung auf eine Schleimhautoberfläche des Säugetiers aufgebracht wird.

24. Zusammensetzung nach Anspruch 23, wobei die Schleimhautoberfläche eine Oberfläche des Magen-Darm-Trakts umfasst.

25. Verwendung eines Phospholipids bei der Herstellung von Mikrokugeln zur Verwendung in der Zusammensetzung nach Anspruch 1.

26. Verwendung eines Phospholipids als Immunostimulans bei der Herstellung von Mikrokugeln für die Verwendung in einem prophylaktischen oder therapeutischen Impfstoff zur Verabreichung auf die Oberfläche einer Schleimhaut.

## Revendications

1. Composition pharmaceutique qui est adaptée pour l'administration sur une surface mucosale, qui comprend des microsphères comprenant
(i) un composé biologiquement actif capable de provoquer une réponse immunitaire ;
(ii) un matériau polymérique capable de former des microsphères et
(iii) un immunostimulant comprenant un phospholipide en une quantité de 0,1 à 20 % m/m et dans lequel le ratio du matériau polymérique sur le phospholipide est de 99:1 à 9:1 % m/m.

2. Composition selon la revendication 1 qui est adaptée pour l'administration orale.

3. Composition selon la revendication 1 ou la revendication 2 **caractérisée en ce que** le matériau polymérique a un poids moléculaire dépassant 94 kDa.

4. Composition selon la revendication 3 **caractérisée en ce que** le matériau polymérique a un poids moléculaire de 100 kDa ou plus.

5. Composition selon l'une quelconque des revendications précédentes **caractérisée en ce que** le matériau polymérique comprend un poly(hydroxy)acide ou un copolymère de cet acide.

6. Composition selon l'une quelconque des revendications précédentes **caractérisée en ce que** le phospholipide est choisi parmi la lécithine ou son précurseur la phosphorylcholine, la distéarylphophatidylcholine (DSPC) et la phosphatidylsérine (PS).

7. Composition selon la revendication 6 **caractérisée en ce que** le phospholipide est la lécithine.

8. Composition selon l'une quelconque des revendications précédentes **caractérisée en ce que** la quantité de phospholipide est de 5 % m/m.

9. Composition selon l'une quelconque des revendications précédentes **caractérisée en ce que** le composé biologiquement actif capable de provoquer une réponse immunitaire chez un animal auquel il est administré comprend un polypeptide ou un acide nucléique.

10. Composition selon la revendication 9 **caractérisée en ce que** ledit agent biologiquement actif comprend l'anatoxine tétanique, l'anatoxine diphtérique, l'antigène protecteur (PA) de *Bacillus anthracis* ou un agent qui est capable de provoquer une réponse immunitaire contre *Yersinia pestis.*

11. Composition selon la revendication 9 ou la revendication 10 qui comprend en outre un adjuvant classique afin d'augmenter la réponse immunitaire au matériau biologiquement actif administré.

12. Composition selon l'une quelconque des revendications précédentes qui comprend en outre un conservateur.

13. Composition selon la revendication 12 **caractérisée en ce que** ledit conservateur est le cétrimonium et est présent en des quantités de 0,1 à 7 % m/v.

14. Composition selon l'une quelconque des revendications précédentes **caractérisée en ce que** les microsphères utilisées dans les compositions peuvent en outre comprendre une protéine de couche S.

15. Composition selon la revendication 14 **caractérisée en ce que** l'agent biologiquement actif est tel que défini dans la revendication 9 et dans laquelle les protéines de couche S sont dérivées d'une bactérie contre laquelle l'agent biologiquement actif produit une réponse immunitaire protectrice.

16. Composition selon l'une quelconque des revendications précédentes **caractérisée en ce que** les microsphères sont en suspension dans un diluant ou un porteur.

17. Méthode pour produire une composition pharmaceutique selon la revendication 1, ladite méthode comprenant l'encapsulation d'un agent biologiquement actif qui est capable de provoquer une réponse immunitaire protectrice, dans un matériau polymérique, en présence d'un phospholipide en une quantité de 0,1 % à 20 % m/m, dans laquelle le ratio du matériau polymérique sur le phospholipide est de 99:1 à 9:1 % m/m.

18. Méthode selon la revendication 17 **caractérisée en ce que** l'agent biologiquement actif, en solution ou en un état lyophilisé de manière adaptée, est mis en suspension ou dissous dans une solution aqueuse du matériau polymérique et du phospholipide, une solution du polymère dans un solvant organique est ajoutée sous agitation vigoureuse, l'émulsion obtenue est versée dans une phase aqueuse secondaire qui contient un matériau polymérique sous agitation vigoureuse, après quoi le solvant organique s'évapore et les microsphères obtenues sont séparées.

19. Méthode selon la revendication 18 **caractérisée en ce que** la phase aqueuse secondaire contient aussi un phospholipide.

20. Méthode selon l'une quelconque des revendications 17 à 19 **caractérisée en ce que** le phospholipide est distribué dans la microsphère.

21. Méthode selon l'une quelconque des revendications 17 à 20 **caractérisée en ce que** la composition comprend un vaccin prophylactique ou thérapeutique.

22. Composition selon l'une quelconque des revendications 1 à 16 à utiliser dans une méthode de protection d'un mammifère contre l'infection par un pathogène.

23. Composition selon la revendication 22 **caractérisée en ce que** la composition est administrée sur une surface mucosale dudit mammifère.

24. Composition selon la revendication 23 **caractérisée en ce que** la surface mucosale comprend une surface gastro-intestinale.

25. Utilisation d'un phospholipide pour la fabrication d'une microsphère à utiliser dans la composition selon la revendication 1.

26. Utilisation d'un phospholipide comme immunostimulant pour la fabrication de microsphères à utiliser dans un vaccin prophylactique ou thérapeutique pour administration sur une surface mucosale.
